# EUROPEAN PATENT SPECIFICATION

(11) **EP 3 703 690 B1**
(45) Date of publication and mention of the grant of the patent: **03.06.2026**
(21) Application number: 18789181.7
(22) Date of filing: 25.10.2018
(51) Int. Cl.: A61K 31/455, A61K 8/67, A61P 17/00, A61Q 17/00

(54) **USE OF NIACINAMIDE FOR MICROBIOME BALANCING**
VERWENDUNG VON NIACINAMID ZUM MIKROBIOMAUSGLEICH
UTILISATION DE NIACINAMIDE POUR L'ÉQUILIBRAGE DE MICROBIOMES

(30) Priority: 30.10.2017 EP 17199211
(43) Date of publication of application: 09.09.2020
(73) Proprietor: Unilever Global IP Limited, Wirral, Merseyside CH62 4ZD (GB); Unilever IP Holdings B.V., 6708 WH Wageningen (NL)
(72) Inventor: CORNMELL, Robert, Joseph, Wirral Merseyside CH63 3JW (GB); MAJUMDAR, Amitabha, Bangalore 560066 (IN); GHOSH, Rimpa, Bangalore 560066 (IN); JAMES, Alexander, Gordon, Sharnbrook Bedfordshire MK44 1LQ (GB)
(74) Representative: Unilever Patent Group
(86) International application number: PCT/EP2018/079304
(87) International publication number: WO 2019/086327

(56) References cited:
- EP-A1- 3 120 850
- WO-A1-2015/013634
- WO-A1-2015/106175
- WO-A1-2015/172801
- WO-A1-2017/202586
- WO-A1-2018/010906
- WO-A2-2015/086843
- DE-A1- 2 160 408
- PIERRE KYME ET AL: "C/EBP&egr; mediates nicotinamide-enhanced clearance of Staphylococcus aureus in mice", JOURNAL OF CLINICAL INVESTIGATION, vol. 122, no. 9, 4 September 2012 (2012-09-04), pages 3316 - 3329, XP055128464, ISSN: 0021-9738, DOI: 10.1172/JCI62070
- SHAHTALEBI M A ET AL: "Preparation and pharmaceutical evaluation of nicotinamide stick for eradication of Staphylococcus epidermidis", vol. 19, no. 7, 1 July 2014 (2014-07-01), pages 654 - 657, XP009504926, ISSN: 1735-1995, Retrieved from the Internet <URL:http://jrms.mui.ac.ir/files/journals/1/articles/9999/public/9999-38732-1-PB.pdf> [retrieved on 20180419]

## Description

### Field of the invention

The invention relates to a new use of niacinamide or its analogue and precursors for microbiome balancing on skin. This has application on skin, scalp against attack by microorganisms.

### Background of the invention

Skin is the primary line of defense that protects the human body from invading pathogens, like viruses and bacteria. As a primary defense organ, the skin tissue always remains in constant contact with the environment and therefore it has to face and resolve threats and challenges from invading pathogens. The exposed skin surface is not only challenged by pathogenic foreign bacteria, but it also remains in contact and interacts with the resident commensal bacteria. In spite of all these challenges from the foreign and the commensal microbes, healthy skin remains infection free and the numbers of the resident microflora remains constant.

An estimated 100 trillion microorganisms inhabit the human body. This includes bacteria, fungi, viruses, protozoa and mites. Skin microbiome refers to this entire population of microbes that are present on skin. Some of the microbes present in the skin considered to be beneficial whereas some of the other microbes that co-habits with the good microbes are detrimental in nature.

One of the known way of achieving skin hygiene by way of application of antimicrobial compositions. There are several antimicrobial compositions disclosed in the art for achieve skin hygiene.

US2008014247A (Lu et al., 2008) discloses a composition having metal containing material, stearic acid and a pharmaceutically acceptable carrier to treat conditions caused by gram-positive, gram-negative, fungal pathogens and/or antibiotic-resistant bacteria. It further provides a method for inhibiting biofilm proliferation. The metal containing material can be silver.

US3050467 B1 (Horowitz et al. 1962) discloses an antimicrobial cleansing composition consisting essentially of a mixture of a water-soluble soap and a silver salt of partially depolymerized alginic acid. The composition provides synergistic antimicrobial activity.

However, the application of antimicrobial composition on the skin also kills those microbes, which are beneficial for skin. This happens because most of the antimicrobial agents cannot work selectively.

The present inventors have surprisingly found that niacinamide when applied on the skin provides effective microbiome balancing by eliminating/lowering bad microbes whereas have no/less effect on the good microbes.

### Summary of the Invention

In a first aspect, the present invention provides non-therapeutic use of niacinamide or its analogue selected from picolinamide, iso-nicotinamide, N-cyclopropyl nicotinamide, N-cyclopentyl nicotinamide, and its precursor tryptophan, for microbiome balancing when applied on an external surface of the human body, wherein the external surface is skin, scalp, or hair;
wherein the microbiome balancing is obtained by selectively reducing the number of harm causing microbes residing on the external surface whereas having less effect on the beneficial microbes,
wherein the ratio of percentage reduction of harm causing microbes to beneficial microbes is in the range of 2:1 to 5:1, and
wherein the harm causing microbe is *S. hominis,* and the beneficial microbe is *S. epidermidis.*

In a second aspect, the present invention provides non-therapeutic use of niacinamide or its analogue selected from picolinamide, iso-nicotinamide, N-cyclopropyl nicotinamide, N-cyclopentyl nicotinamide, and its precursor tryptophan, in a topical composition as a microbiome balancing agent,
wherein the microbiome balancing is obtained by selectively reducing the number of harm causing microbes residing on an external surface of the human body, whereas having less effect on the beneficial microbes,
wherein the external surface is skin, scalp, or hair
wherein the ratio of percentage reduction of harm causing microbes to beneficial microbes is in the range of 2:1 to 5:1, and
wherein the harm causing microbe is *S. hominis,* and the beneficial microbe is *S. epidermidis.*

In a third aspect, the present invention provides niacinamide or its analogue selected from picolinamide, iso-nicotinamide, N-cyclopropyl nicotinamide, N-cyclopentyl nicotinamide, and its precursor tryptophan, or a composition comprising niacinamide or its analogue selected from picolinamide, iso-nicotinamide, N-cyclopropyl nicotinamide, N-cyclopentyl nicotinamide, and its precursor tryptophan, for therapeutic use in microbiome balancing when applied on an external surface of the human body, wherein the external surface is skin, scalp, or hair,
wherein the microbiome balancing is obtained by selectively reducing the number of harm causing microbes residing on the external surface whereas having less effect on the beneficial microbes; and
wherein the ratio of percentage reduction of harm causing microbes to beneficial microbes is in the range of 2:1 to 5:1, and
wherein the harm causing microbe is *S. hominis,* and the beneficial microbe is *S. epidermidis.*

In a fourth aspect the present invention provides non-therapeutic use of niacinamide or its analogue selected from picolinamide, iso-nicotinamide, N-cyclopropyl nicotinamide, N-cyclopentyl nicotinamide, and its precursor tryptophan, for malodour reduction when applied on an external surface of the human body,
wherein the external surface is skin, scalp, or hair,
wherein the malodour reduction is achieved by way of microbiome balancing which is obtained by selectively reducing the number of harm causing microbes residing on the external surface whereas having less effect on the beneficial microbes; and
wherein the ratio of percentage reduction of harm causing microbes to beneficial microbes is in the range of 2:1 to 5:1, and
wherein the harm causing microbe is *S. hominis,* and the beneficial microbe is *S. epidermidis.*

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed Description of the Invention

"Skin" as used herein, is meant to include the external surface of mammals, especially humans and includes skin, scalp and hair. The use could be by way of incorporating niacinamide or its analogue and precursors in a leave-on or in a rinse off product, and includes any product applied to a human body primarily for lightening skin but may also improve appearance, cleansing, odor control or general aesthetics. The use is preferably by way of incorporation in a leave-on composition. The composition can be in the form of a liquid, lotion, cream, foam, scrub, gel, soap bar or toner, or applied with an implement or via a face mask, pad or patch. Non-limiting examples of such compositions include leave-on skin lotions and creams, shampoos, conditioners, shower gels, toilet bars, antiperspirants, deodorants, depilatories, lipsticks, foundations, mascara, sunless tanners and sunscreen lotions.

Niacinamide has the structure as given below

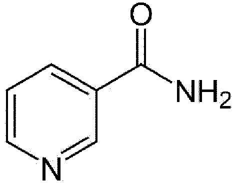

Niacinamide is also known as nicotinamide or Vitamin B3.

The present invention provides non-therapeutic use of niacinamide or its analogue and precursor for microbiome balancing when applied on an external surface of the human body, as defined in the claims.

The analogues of niacinamide are selected from picolinamide, iso-nicotinamide, N-cyclopropyl nicotinamide, N-cyclopentyl nicotinamide. The precursor of niacinamide is tryptophan.

The **phrase "microbiome balancing" used** herein means selectively reducing the harm causing microbes existing on the skin whereas having less effect on the beneficial microbes. The ratio of percentage reduction of harm causing microbes to beneficial microbes is in the range of 2:1 to 5:1. The above ratios ensure that there are high numbers of the beneficial microorganisms left on the skin.

The microbiome balancing is obtained by substantially reducing *S. hominis.* whereas having less detrimental effect on the beneficial microorganism *S. epidermidis.* Beneficial microorganism like *S. epidermidis* is proven to have a beneficial role in immune defense system of human skin.

Niacinamide is known as an efficient skin lightening agent and used in many cosmetic formulations. Another known use of niacinamide as disclosed in WO 2015/172801 (Unilever, 2015) is for inducing generation of antimicrobial peptides (AMP) on the skin.

Preferably the external surface comprises keratinocytes e.g. human skin, scalp etc.

The use of niacinamide as per the present invention is non-therapeutic,e.g. for cosmetic application. Cosmetic use preferably means the use for prevention rather than cure. E.g. Some bacteria like *Escherichia coli (E. coli)* and *Staphylococcus aureus* (*S. aureus*) are commonly found on the human skin. These bacteria per se do not trigger a pathogenic effect whilst commonly present on the skin. However, when they enter the human body through cuts on the skin and through acts like ingestion, these bacteria become pathogenic. Additionally, bacteria like *S. hominis* does not *per se* have any harmful effect on skin on its own. However, it reacts with skin biproducts like sebum to produce substance that cause foul smell, which is undesirable. For the purpose and definition of the invention *S. hominis* is also considered to be a harmful microorganism. Therefore, keeping the external surface of the body like e.g. hand and scalp, free of these microorganisms (bacteria) helps in preventing them from entering the human body or causing malodor, thereby achieving the desired hygiene.

The present invention also provides non-therapeutic use of niacinamide in a topical composition as a microbiome balancing agent as defined in the claims.

The present invention also provides non-therapeutic use of niacinamide or its analogue and precursors for malodour reduction when applied on an external surface of the human body, wherein the malodour reduction is achieved by way of microbiome balancing as defined in the claims.

Malodour is generally caused by the reaction of skin biproduct e.g. sebum with some harmful microorganism like *S. hominis.* One of the advantages of present invention is that by way of using niacinamide or its analogue and precursors the malodour is reduced. This is achieved by selectively reducing *S. hominis* in a much higher extent than *S. epidermidis* thereby maintaining a high level of beneficial microorganisms on the surface.

The composition in which niacinamide or its analogue and precursors is used as per the present invention may comprise the following preferred features. Niacinamide or its analogue and precursors is preferably present in 0.1 to 5%, more preferably 0.5 to 5%, furthermore preferably 0.5 to 3%, and optimally 1.0 to 3.0% by weight of the composition. The composition preferably comprises a cosmetically acceptable base. The cosmetically acceptable base is preferably a cream, lotion, gel or emulsion.

Personal care compositions may be prepared using different cosmetically acceptable emulsifying or non-emulsifying systems and vehicles. Preferred cosmetically acceptable bases comprise 1 to 25% fatty acid. A further preferred aspect provides for inclusion of 0.1 to 10% soap. A highly suitable base is a cream. Vanishing creams are especially preferred. Vanishing cream bases generally comprise 5 to 25% w/w fatty acid and 0.1 to 10% w/w soap. Vanishing cream base gives a highly appreciated matty feel to the skin. C₁₂ to C₂₀ fatty acids are especially preferred in vanishing cream bases, furthermore preferred being C₁₄ to C₁₈ fatty acids. The most preferred fatty acid is stearic acid. The fatty acid may also be a mixture of palmitic and stearic acid. The fatty acid in the composition is more preferably present in an amount in the range of 5 to 20% w/w of the composition. Soaps in the vanishing cream base include alkali metal salt of fatty acids, like sodium or potassium salts, most preferred being potassium stearate. The soap in the vanishing cream base is generally present in an amount in the range of 0.1 to 10%, more preferably 0.1 to 3% w/w of the composition. The personal care composition when formulated as a vanishing cream preferably comprises 60 to 85%, more preferably 65 to
80% w/w water. Water is generally present in many compositions prepared as per the invention and is generally present in 40 to 85% by weight of the composition.

The composition of the invention may comprise other optional ingredients like skin lightening agents, and one or more UV sunscreens. The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition may optionally be delivered as a deodorant. By a deodorant is meant a product in the stick, roll-on, or propellant medium which is used for personal deodorant benefit e.g. application in the under-arm area which may or may not contain anti-perspirant actives.

Deodorant compositions can generally be in the form of firm solids, soft solids, gels, creams, and liquids and are dispensed using applicators appropriate to the physical characteristics of the composition.

The compositions may comprise a wide range of other optional components. The

CTFA Cosmetic Ingredient Handbook, Second Edition,1992,
describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents,
pH adjusters, preservatives, natural extracts, essential oils, skin sensates, skin soothing agents, and skin healing agents. The composition is formulated in any known format like shampoo, leave-on creams, lotions, tonic or serum more preferred formats being shampoos, creams or lotions.

The invention is now further described by way of the following non-limiting examples.

### Examples

To check the microbiome balancing efficacy of niacinamide roll-on compositions were prepared as detailed in Table 1 by methods known in the art.

**Table 1**

| **Component** | | **Example 1** | **Control** |
|---|---|---|---|
| **RM name** | **Hierarchy name** | **Wt %** | |
| Niacinamide | Niacinamide | 5.00 | -- |
| Sunflower seed oil | *Helianthus anuus* seed oil | 4.00 | 4.00 |
| Glycerin | Glycerol | 4.00 | 4.00 |
| Steareth-2 | Ethoxylated alcohol C18/E2 | 2.60 | 2.60 |
| Steareth-20 | Ethoxylated alcohol C18/E20 | 0.60 | 0.60 |
| Phenoxyethanol | 2-phenoxyethanol | 0.40 | 0.40 |
| Glycacil L* | 3-iodo-2-propynyl butyl carbamate, PEG laurate and PEG dilaurate | 0.07 | 0.07 |
| Water | Water | To 100 | To 100 |

A panel of 50 female panelists was employed. At the start of the test, panelists were washed with unfragranced soap and different treatments applied to each axilla using a conventional roll-on applicator with a target dose of 0.30g (+/- 30mg) via strokes of the roll-on. Application was randomised to take into account any left/right bias. Panelists were instructed not to consume spicy food or alcohol, and not to wash under their own axillae, during the duration of the test.

Microbiological sampling was carried out at the 24 hours timepoint, and was conducted in order to assess the numbers of the main bacterial types resident in the axillae, namely staphylococci (*S. epidermidis* and *S*. *hominis*).. Each panellist provided a single sample from both axillae at one timepoint on one of the test days using the scrub cup technique as described by Taylor et al. (International Journal of Cosmetic Science, Vol 25, 2003, pp 137-145). Samples were diluted and dispensed onto agar plates containing culture media selective for staphylococci, using an automatic spiral plater. After incubation, plates were enumerated using a colony counter and the data used to calculate log10 transformed colony-forming units per square cm skin (Log CFU / cm2). Bacterial numbers were averaged across the four daily sample collections, and the data analysed statistically.

The results are summarized below in Table 2.

**Table 2:**

| Type of bacteria | Control (ng/µL) | Example 1 (ng/µL) | Change from control | % reduction over control |
|---|---|---|---|---|
| S. epidermidis | 2.34 X10⁻² | 2.19 X10⁻² | 0.14 X10⁻² | 5.9 % |
| S. hominis | 3.99 X 10⁻³ | 2.93 X10 ⁻³ | 1.06 X10 ⁻³ | 26.5 % |

The data were found to be statistically significant.

From the above table it is evident that the composition of Example 1 which comprises 5% B3 is able to substantially reduce the harm causing microbe *S.hominis* (known for causing malodour in axillae). At the same time, it is also evident that the same composition (Example 1) does not effectively reduce the good bacteria *S. epidermidis.* From the data, it can be seen that, for *S. epidermidis* the reduction is only 5.9% whereas for S. hominis, the reduction is 26.5%. As can be seen from the above data the ratio of percentage reduction of harm causing microbes (*S. hominis*) and beneficial microbes (*S. epidermidis*) is 26.5 : 5.9 i.e. 4.5 :1.Therefore it is clear from the data that niacinamide indeed does microbiome balancing on the skin i.e. selectively reduce the number of harm causing microbes(*S*. *hominis*) whereas it has less effect on the good microbes (*S. epidermidis*).

## Claims

1. Non-therapeutic use of niacinamide or its analogue selected from picolinamide, iso-nicotinamide, N-cyclopropyl nicotinamide, N-cyclopentyl nicotinamide, and its precursor tryptophan, for microbiome balancing when applied on an external surface of the human body, wherein the external surface is skin, scalp, or hair;
wherein the microbiome balancing is obtained by selectively reducing the number of harm causing microbes residing on the external surface whereas having less effect on the beneficial microbes,
wherein the ratio of percentage reduction of harm causing microbes to beneficial microbes is in the range of 2:1 to 5:1, and
wherein the harm causing microbe is *S. hominis,* and the beneficial microbe is *S. epidermidis*

2. Use as claimed in any one of the preceding claims wherein said external surface comprises keratinocytes.

3. Use as claimed in any one of the preceding claims wherein said external surface includes skin or scalp.

4. Non-therapeutic use of niacinamide or its analogue selected from picolinamide, iso-nicotinamide, N-cyclopropyl nicotinamide, N-cyclopentyl nicotinamide, and its precursor tryptophan, in a topical composition as a microbiome balancing agent,
wherein the microbiome balancing is obtained by selectively reducing the number of harm causing microbes residing on an external surface of the human body, whereas having less effect on the beneficial microbes,
wherein the external surface is skin, scalp, or hair
wherein the ratio of percentage reduction of harm causing microbes to beneficial microbes is in the range of 2:1 to 5:1, and
wherein the harm causing microbe is *S. hominis,* and the beneficial microbe is *S. epidermidis*

5. Niacinamide or its analogue selected from picolinamide, iso-nicotinamide, N-cyclopropyl nicotinamide, N-cyclopentyl nicotinamide, and its precursor tryptophan, or a composition comprising niacinamide or its analogue selected from picolinamide, iso-nicotinamide, N-cyclopropyl nicotinamide, N-cyclopentyl nicotinamide, and its precursor tryptophan, for therapeutic use in microbiome balancing when applied on an external surface of the human body,
wherein the external surface is skin, scalp, or hair,
wherein the microbiome balancing is obtained by selectively reducing the number of harm causing microbes residing on the external surface whereas having less effect on the beneficial microbes; and
wherein the ratio of percentage reduction of harm causing microbes to beneficial microbes is in the range of 2:1 to 5:1, and
wherein the harm causing microbe is *S. hominis,* and the beneficial microbe is *S. epidermidis*

6. Non-therapeutic use of niacinamide or its analogue selected from picolinamide, iso-nicotinamide, N-cyclopropyl nicotinamide, N-cyclopentyl nicotinamide, and its precursor tryptophan, for malodour reduction when applied on an external surface of the human body,
wherein the external surface is skin, scalp, or hair,
wherein the malodour reduction is achieved by way of microbiome balancing which is obtained by selectively reducing the number of harm causing microbes residing on the external surface whereas having less effect on the beneficial microbes; and wherein the ratio of percentage reduction of harm causing microbes to beneficial microbes is in the range of 2:1 to 5:1, and
wherein the harm causing microbe is *S. hominis,* and the beneficial microbe is *S. epidermidis*

## Patentansprüche

1. Nicht-therapeutische Verwendung von Niacinamid oder seinen Analoga, ausgewählt unter Picolinamid, Iso-Nicotinamid, N-Cyclopropylnicotinamid, N-Cyclopentylnicotinamid und seinem Vorläufer Tryptophan, zum Mikrobiomausgleich bei Anwendung auf einer äußeren Oberfläche des menschlichen Körpers, wobei die äußere Oberfläche Haut, Kopfhaut oder Haar ist; wobei der Mikrobiomausgleich durch selektive Verringerung der Anzahl schädlicher Mikroben, die sich auf der äußeren Oberfläche befinden, erreicht wird, während diese weniger Auswirkung auf die nützlichen Mikroben hat,
wobei das Verhältnis der prozentualen Verringerung der schädlichen Mikroben zu den nützlichen Mikroben im Bereich von 2:1 bis 5:1 liegt, und
wobei die schädliche Mikrobe *S. hominis* und die nützliche Mikrobe *S. epidermidis* ist.

2. Verwendung nach irgendeinem der vorhergehenden Ansprüche, wobei die äußere Oberfläche Keratinozyten umfasst.

3. Verwendung nach irgendeinem der vorhergehenden Ansprüche, wobei die äußere Oberfläche Haut oder Kopfhaut umfasst.

4. Nicht-therapeutische Verwendung von Niacinamid oder seinen Analoga, ausgewählt unter Picolinamid, Iso-Nicotinamid, N-Cyclopropylnicotinamid, N-Cyclopentylnicotinamid und seinem Vorläufer Tryptophan, in einer topischen Zusammensetzung als mikrobiomausgleichendes Mittel,
wobei der Mikrobiomausgleich durch selektive Verringerung der Anzahl schädlicher Mikroben, die sich auf der äußeren Oberfläche des menschlichen Körpers befinden, erreicht wird, während diese weniger Auswirkung auf die nützlichen Mikroben hat,
wobei die äußere Oberfläche Haut, Kopfhaut oder Haar ist,
wobei das Verhältnis der prozentualen Verringerung der schädlichen Mikroben zu den nützlichen Mikroben im Bereich von 2:1 bis 5:1 liegt, und
wobei die schädliche Mikrobe *S. hominis* und die nützliche Mikrobe *S. epidermidis* ist.

5. Niacinamid oder seine Analoga, ausgewählt unter Picolinamid, Iso-Nicotinamid, N-Cyclopropylnicotinamid, N-Cyclopentylnicotinamid und seinem Vorläufer Tryptophan, oder eine Zusammensetzung, umfassend Niacinamid oder seine Analoga, ausgewählt unter Picolinamid, Iso-Nicotinamid, N-Cyclopropylnicotinamid, N-Cyclopentylnicotinamid und seinem Vorläufer Tryptophan, zur therapeutischen Verwendung beim Mikrobiomausgleich bei Anwendung auf einer äußeren Oberfläche des menschlichen Körpers,
wobei die äußere Oberfläche Haut, Kopfhaut oder Haar ist,
wobei der Mikrobiomausgleich durch selektive Verringerung der Anzahl schädlicher Mikroben, die sich auf der äußeren Oberfläche befinden, erreicht wird, während diese weniger Auswirkung auf die nützlichen Mikroben hat,
wobei das Verhältnis der prozentualen Verringerung der schädlichen Mikroben zu den nützlichen Mikroben im Bereich von 2:1 bis 5:1 liegt, und
wobei die schädliche Mikrobe *S. hominis* und die nützliche Mikrobe *S. epidermidis* ist.

6. Nicht-therapeutische Verwendung von Niacinamid oder seinen Analoga, ausgewählt unter Picolinamid, Iso-Nicotinamid, N-Cyclopropylnicotinamid, N-Cyclopentylnicotinamid und seinem Vorläufer Tryptophan, zur Reduzierung unangenehmen Geruchs bei Anwendung auf einer äußeren Oberfläche des menschlichen Körpers,
wobei die äußere Oberfläche Haut, Kopfhaut oder Haar ist,
wobei die Reduzierung des unangenehmen Geruchs durch Mikrobiomausgleich erreicht wird,
wobei der Mikrobiomausgleich durch selektive Verringerung der Anzahl schädlicher Mikroben, die sich auf der äußeren Oberfläche befinden, erreicht wird, während diese weniger Auswirkung auf die nützlichen Mikroben hat; und
wobei das Verhältnis der prozentualen Verringerung der schädlichen Mikroben zu den nützlichen Mikroben im Bereich von 2:1 bis 5:1 liegt, und
wobei die schädliche Mikrobe *S. hominis* und die nützliche Mikrobe *S. epidermidis* ist.

## Revendications

1. Utilisation non thérapeutique de niacinamide ou de son analogue choisi parmi le picolinamide, l'iso-nicotinamide, le N-cyclopropyl-nicotinamide, le N-cyclopentyl-nicotinamide, et son précurseur tryptophane, pour l'équilibrage du microbiote, quand il est appliqué sur une surface externe du corps humain, dans laquelle la surface externe est la peau, le cuir chevelu, ou les cheveux ;
dans laquelle l'équilibrage du microbiote est obtenu par réduction sélective du nombre de microbes pathogènes résidant sur la surface externe cependant que l'effet sur les microbes bénéfiques est moindre,
dans laquelle le rapport de la réduction en pourcentage des microbes pathogènes aux microbes bénéfiques est situé dans la plage allant de 2/1 à 5/1, et
dans laquelle le microbe pathogène est *S. hominis,* et le microbe bénéfique est *S. epidermidis.*

2. Utilisation selon l'une quelconque des revendications précédentes, dans laquelle ladite surface externe comprend des kératinocytes.

3. Utilisation selon l'une quelconque des revendications précédentes, dans laquelle ladite surface externe englobe la peau ou le cuir chevelu.

4. Utilisation non thérapeutique de niacinamide ou de son analogue choisi parmi le picolinamide, l'iso-nicotinamide, le N-cyclopropyl-nicotinamide, le N-cyclopentyl-nicotinamide, et son précurseur tryptophane, dans une composition à usage topique en tant qu'agent équilibrant le microbiote,
dans laquelle l'équilibrage du microbiote est obtenu par réduction sélective du nombre de microbes pathogènes résidant sur une surface externe du corps humain cependant que l'effet sur les microbes bénéfiques est moindre,
dans laquelle la surface externe est la peau, le cuir chevelu, ou les cheveux,
dans laquelle le rapport de la réduction en pourcentage des microbes pathogènes aux microbes bénéfiques est situé dans la plage allant de 2/1 à 5/1, et
dans laquelle le microbe pathogène est *S. hominis,* et le microbe bénéfique est *S. epidermidis.*

5. Niacinamide ou son analogue choisi parmi le picolinamide, l'iso-nicotinamide, le N-cyclopropyl-nicotinamide, le N-cyclopentyl-nicotinamide, et son précurseur tryptophane, ou composition comprenant du niacinamide ou son analogue choisi parmi le picolinamide, l'iso-nicotinamide, le N-cyclopropyl-nicotinamide, le N-cyclopentyl-nicotinamide, et son précurseur tryptophane, pour une utilisation thérapeutique dans l'équilibrage du microbiote, quand il est appliqué sur une surface externe du corps humain,
dans lequel la surface externe est la peau, le cuir chevelu, ou les cheveux ;
dans lequel l'équilibrage du microbiote est obtenu par réduction sélective du nombre de microbes pathogènes résidant sur une surface externe du corps humain cependant que l'effet sur les microbes bénéfiques est moindre ; et
dans lequel le rapport de la réduction en pourcentage des microbes pathogènes aux microbes bénéfiques est situé dans la plage allant de 2/1 à 5/1, et
dans lequel le microbe pathogène est *S. hominis,* et le microbe bénéfique est *S. epidermidis.*

6. Utilisation non thérapeutique de niacinamide ou de son analogue choisi parmi le picolinamide, l'iso-nicotinamide, le N-cyclopropyl-nicotinamide, le N-cyclopentyl-nicotinamide, et son précurseur tryptophane, pour la réduction des mauvaises odeurs, quand il est appliqué sur une surface externe du corps humain,
dans laquelle la surface externe est la peau, le cuir chevelu, ou les cheveux,
dans laquelle la réduction des mauvaises odeurs est réalisée au moyen d'un équilibrage du microbiote qui est obtenu par réduction sélective du nombre de microbes pathogènes résidant sur une surface externe du corps humain cependant que l'effet sur les microbes bénéfiques est moindre, et
dans laquelle le rapport de la réduction en pourcentage des microbes pathogènes aux microbes bénéfiques est situé dans la plage allant de 2/1 à 5/1, et
dans laquelle le microbe pathogène est *S. hominis,* et le microbe bénéfique est *S. epidermidis.*
